# EUROPEAN PATENT APPLICATION

(11) **EP 3 975 193 A1**
(43) Date of publication of application: **30.03.2022**
(21) Application number: 20197993.7
(22) Date of filing: 24.09.2020
(51) Int. Cl.: G16H 20/40, G16H 50/70, G16H 50/20, A61B 34/00

(54) **AIDING DECISION MAKING IN SELECTING AN IMPLANT TREATMENT**

(71) Applicant: Koninklijke Philips N.V., 5656 AG Eindhoven (NL)
(72) Inventor: WIRTH, Christoph Tobias, 5656 AE Eindhoven (NL); VAN DE CRAEN, Dieter Maria Alfons, 5656 AE Eindhoven (NL)
(74) Representative: Philips Intellectual Property & Standards

(57) **Abstract**

A system and computer-implemented method are defined for aiding decision making in selecting an implant treatment for a subject are provided. By exploiting a plurality of outcome prediction models based on historic treatment data, predicted treatment outcomes can be generated for a large number of different implant treatment types. These predicted treatment outcomes can then be generated based on subject specific pre-implantation baseline data, to give predicted treatment outcomes specific to the subject. By outputting the predicted subject outcomes for a large number of implant treatment types, the subject, in consultation with a doctor and/or surgeon, may make a more informed decision over a wide range of implant treatment types.

## Description

### FIELD OF THE INVENTION

The present invention is generally related to implant treatments and, particularly, to aiding decision making in selecting an implant treatment for a subject.

### BACKGROUND OF THE INVENTION

With an ageing population, the number of orthopedic prosthesis procedures, such as total hip and knee replacements, will continue to increase. Today, orthopedic issues are one of the most common reasons people seek medical care, with one in seven Americans reporting an orthopedic impairment.

In treating a subject, a surgeon needs to make a number of decisions including choosing from a variety of implant features, and the surgical technique to employ. The number of options with respect to these implant features and surgical techniques can be vast. As a result, studies have shown clear differences in the preferences of surgeons. For example, some surgeons choose to use a preferred implant vendor's newest models as soon as they become available. Implant vendors typically demonstrate the advantages of their latest implants in laboratory simulation studies. However, actual outcomes such as durability and complication rates can only be established once the implant has been in use for several years. This can result in unforeseen issues, such as those demonstrated by the early failures in metal-on-metal hip replacements. Such challenges are also present in several other areas that make use of implantable devices, such as stents and defibrillators.

It is believed that, with increasing accountability for hospitals and health care systems, there will be a drive towards a more evidence based decision making system, rather than relying on the surgeon's personal experiences and beliefs.

To aid decision-making, there now exist national registries, such as the Dutch Arthroplasty Register (LROI), which contain information on orthopedic prosthesis procedures. Such registries typically collect data on implant characteristics, surgical techniques, survival of implants, subject characteristics, and subject experiences.

### SUMMARY OF THE INVENTION

The invention is defined by the claims.

According to examples in accordance with an aspect of the invention, there is provided a system for aiding decision making in selecting an implant treatment for a subject, comprising:
a modelling module configured to obtain a plurality of outcome prediction models, wherein each outcome prediction model is based on historic treatment data of a plurality of different implant treatment types, wherein each outcome prediction model is associated with one of the plurality of different implant treatment types, and wherein each outcome prediction model is used to predict treatment outcomes due to the associated implant treatment type;
a data processing module configured to receive pre-implantation baseline data of the subject, and further configured to determine predicted subject treatment outcomes from each outcome prediction model based on the pre-implantation baseline data of the subject; and
an interface module configured to output the predicted subject treatment outcomes of the subject.

Proposed concepts thus aim to provide schemes, solutions, concepts, designs, methods and systems pertaining to aiding decision making in selecting an implant treatment for a subject. In particular, embodiments of the invention propose generating predicted treatment outcomes for many different implant treatment types based on subject's pre-implantation baseline data. Implant treatment types not yielding desired predicted treatment outcomes may then be filtered out based on other preferences set by the subject. In this way, outcomes for a number of treatments may be predicted for a given subject.

It is proposed that outcome prediction modules may be associated with respective implant treatment types, such that each outcome prediction model can be used to predict treatment outcomes for the associated implant treatment type. Pre-implantation baseline data of the subject may then be obtained, and this data then used to generate the predicted treatment outcomes of the outcome prediction modules. In this way, predicted subject treatment outcomes can be obtained. Embodiments may therefore output predicted subject treatment outcomes which are relevant to the subject, each of which are associated with a different implant treatment type. These predicted subject treatment outcomes may aid decision making as to which implant treatment type may be most suitable for the given subj ect.

Typically, decision making in selecting an implant treatment for a subject is based on a surgeon's own experiences and beliefs. As there are many different implant treatment types to choose from, surgeons typically do not have direct experience with each implant treatment type. As a result, surgeons typically base predicted outcomes on personal experience and on a small amount of pre-implantation baseline data of the subject. It has been realized that by obtaining outcome prediction models based on historic treatment data, a larger number of implant treatment types may be assessed based on their suitability for a given subject, thus aiding decision making in selecting an implant treatment for a subject.

Embodiments may therefore provide the advantage that decision making in selecting an implant treatment can be improved. For example, embodiments may enable a large number of predicted treatment outcomes, each being associated with an implant treatment type, to be compared quickly and/or easily In addition, because results may be generated based on pre-implantation baseline data of the subject, only the predictions most relevant to a given subject may be compared, thus making a decision process easier.

In other words, embodiments propose to generate predicted treatment outcomes for many different treatments using a plurality of outcome prediction models. The predicted treatment outcomes may aid clinical decision making. Accordingly, embodiments may be used in relation to implant treatment selection so as support a medical professional when selecting treatment for a subject. Such embodiments may also support clinical planning. Improved Clinical Decision Support (CDS) may therefore be provided by proposed concepts.

The historic treatment data may, for example, comprise a plurality of pre-implantation baseline data and a plurality of post-implantation outcome data. The modelling module may for example, further comprise a classification module configured to categorise the plurality of pre-implantation baseline data into a plurality of pre-implantation baseline classes, and to categorise the plurality of post-implantation outcome data into a plurality of post-implantation outcome classes.

The historic treatment data used to create the outcome prediction models comprises both pre-implantation baseline data of historic subjects, and post-implantation outcome data of historic subjects. This data may be obtained from, for example, national registries, and may therefore already be available in large quantities.

The pre-implantation baseline data may be grouped into classes based on factors such as diagnosis, pain, functionality and demographics. Such an approach provides many classes of subjects pre-operation, simplifying the interpretation of the data as there may be many thousands of different historic subjects. Post-implantation outcome data may be grouped into classes based on factors such as pain, functionality, complication rate and reoperation rate, for example. Post-implantation data grouped into classes may provide the benefit of simplifying the ranking of outcomes which are preferable for a subject.

The classification module may be configured, for example, to categorise the plurality of pre-implantation baseline data into the plurality of pre-implantation baseline classes, and to categorise the plurality of post-implantation outcome data into the plurality of post-implantation outcome classes, according to at least two variables, and the at least two variables may comprise a pain factor and a functionality factor. This may further clarify that both the pre-implantation baseline data, and post-implantation outcome data can be grouped according to pain and functionality specifically. Embodiments may thus allow the grouping of subjects pre-implantation into groups with similar pain and functionality, and the grouping of subjects post-implantation into groups with similar pain and functionality.

Obtaining the plurality of outcome prediction models may comprise, for example, calculating a plurality of probabilities that a subject associated with each of the pre-implantation baseline classes will be associated with each of the post-implantation outcome classes after receiving each implant treatment type.

The categorisation into pre-implantation and post-implantation classes may be utilised by calculating probabilities of transitioning from one class in the pre-implantation category, to one class in the post-implantation category due to each implant type. This may provide a clear way to interpret which treatment type may be preferable, as probabilities are generally easy for a given subject and surgeon to interpret. By doing this for all possible combinations, an outcome prediction model for each implant treatment type may be generated in the form of a probability mapping.

Calculating the plurality of probabilities may, for example, be based on a logistic regression of the historic treatment data.

The data processing module may be configured, for example, to identify the pre-implantation baseline class which most closely describes the subject based on the pre-implantation baseline data of the subject, and the data processing module may be further configured to communicate only the post-implantation outcome classes and associated probabilities which are associated with the identified pre-implantation baseline class to the interface module. In this way, the subject may be matched with one of the pre-implantation baseline classes, based on pre-implantation baseline data of the subject. This matching can then be utilised in order to derive results from the outcome prediction models, meaning that predicted subject treatment outcomes can be obtained.

The interface module may be configured, for example, to output each of the implant treatment types, and the probability that the subject will be associated with each post-implantation outcome class due to each implant treatment type.

The interface module may display all of the implant treatment types, and the probabilities that the subject will be associated with each of the post-implantation outcome classes if the implantable device is chosen. Embodiments may therefore allow the subject and/or surgeon to observe the full range of implant treatment types available and the likelihood of a wide range of outcomes.

The interface module may be further configured, for example, to obtain a subject preference profile of the subject, to determine one or more preferred implant treatment types based on a comparison between the subject preference profile and the probability that the subject will be associated with each post-implantation outcome class due to each of the implant treatment types, and to output the one or more preferred implant treatment types and the probability that the subject will be associated with each post-implantation outcome class due to each of the one or more preferred implant treatment types.

Alternatively, by obtaining a subject preference profile of the subject, only a selection of implant treatment types with the highest probability of being associated with outcomes preferable to the subject may be presented. By doing this, the outputted information may be further simplified (and thus easier to process).

The plurality of implant treatment types may be associated with, for example, one or more implant variables. By way of example, the one or more implant variables may include a surgical approach, and implant type related variables.

Each of the plurality of implant treatment types may be further associated, for example, with one or more treatment variables. The one or more treatment variables may include healthcare system related variables, and surgeon related variables.

The pre-implantation baseline data may, for example, include one or more of a diagnosis, a pain factor; a functionality factor, subject lifestyle data, and subject demographic data.

The post-implantation outcome data may, for example, include one or more of a pain factor, a functionality factor, a quality of life factor, a complication rate, a reoperation rate, and a 30 day readmission rate. The implant treatment type may be, for example, an implantable device for a hip replacement surgery, and the historic treatment data may comprise data relating to historic hip replacement surgeries.

According to examples in accordance with another aspect of the invention, there is provided a computer-implemented method for aiding decision making in selecting an implant treatment for a subject, comprising:
obtaining a plurality of outcome prediction models, wherein each outcome prediction model is based on historic treatment data of a plurality of different implant treatment types, wherein each outcome prediction model is associated with one of the plurality of different implant treatment types, and wherein each outcome prediction model is used to predict treatment outcomes due to the associated implant treatment type;
receiving pre-implantation baseline data of the subject;
determining predicted subject treatment outcomes from each outcome prediction model based on the pre-implantation baseline data of the subject; and
outputting the subject predicted treatment outcomes of the subject.

According to examples in accordance with another aspect of the invention, there is provided a computer program comprising computer program code means adapted, when said computer program is run on a computer, to implement a method according to an embodiment.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 a simplified diagram of an exemplary embodiment of a system adapted for aiding decision making in selecting an implant treatment for a subject;
Fig. 2 shows a schematic of historic treatment data, and implant and treatment data;
Fig. 3 shows an example of a user interface to represent the output of a system adapted for aiding decision making in selecting an implant treatment for a subject;
Fig. 4 is a flowchart depicting a computer implemented method for aiding decision making in selecting an implant treatment for a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to aiding decision making in selecting an implant treatment for a subject. According to proposed concepts, a number of possible solutions may be implemented separately or jointly. That is, although these possible solutions may be described below separately, two or more of these possible solutions may be implemented in one combination or another.

The invention proposes concepts for aiding decision making in selecting an implant treatment for a subject, which may provide the subject and/or a surgeon with greater information surrounding the likelihood of various outcomes due to a selection of an implant treatment. In particular, embodiments may provide a method and/or system which predicts outcomes related to implant treatments (such as total hip replacements), and uses these predictions to support the decision making for an implant treatment to be used.

In particular, proposed concepts may provide am approach to generating and supplying useful treatment outcome predictions for a subject. Accordingly, embodiments may be used in relation to implant treatment selection and/or provide improved Clinical Decision Support (CDS).

A first element of the system may support the collection of historic treatment data. Patient-reported, clinical and administrative data may be collected at different points in time. The historic treatment data is stored in such a way as to abide by the necessary legal and privacy requirements, while making the data available for the modelling module.

A key element of a proposed embodiment is the use of outcome prediction models, which enable predictions of outcomes at defined time points given a set of subject pre-implantation baseline data. For a selected number of key outcomes such as pain level, quality of life, functional status, risk of revision, expected implant survival predictive models may be built and regularly updated for each implant treatment type.

Another key element of an embodiment is the use of the output(s) of the outcome prediction models to list the most promising options based on a subject preference profile. This may include the maximization or minimization of a certain outcome, or finding a balance between all outcomes. This enables the surgeon, when discussing the patient's preferences, concerns, and expectations, to offer the most optimal personalized solution based on actual, subject-specific, predicted outcomes.

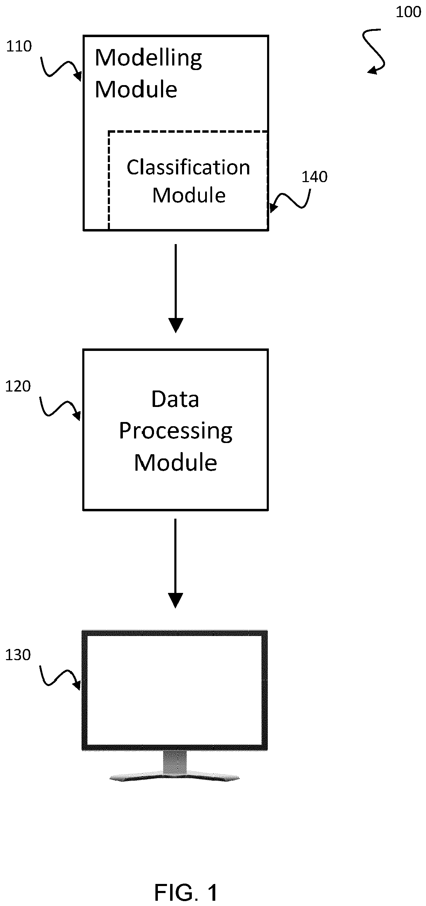

Referring now to Fig. 1, there is depicted a simplified diagram of a system 100 for aiding decision making in selecting an implant treatment for a subject. Specifically, the system comprises a modelling module 110, a data processing module 120, and an interface module 130 in accordance with the invention.

The modelling module 110 is configured to obtain a plurality of outcome prediction models. These outcome prediction models are based on historic treatment data, while each outcome prediction module is associated with one of the plurality of different implant treatment types, and each one is used to predict the treatment outcomes due to the associated implant treatment type. In other words, an outcome prediction model can be used to predict the result of an implant treatment on a subject, and the plurality of outcome prediction models may provide a plurality of predicted outcomes, each related to one of the plurality of different treatment types.

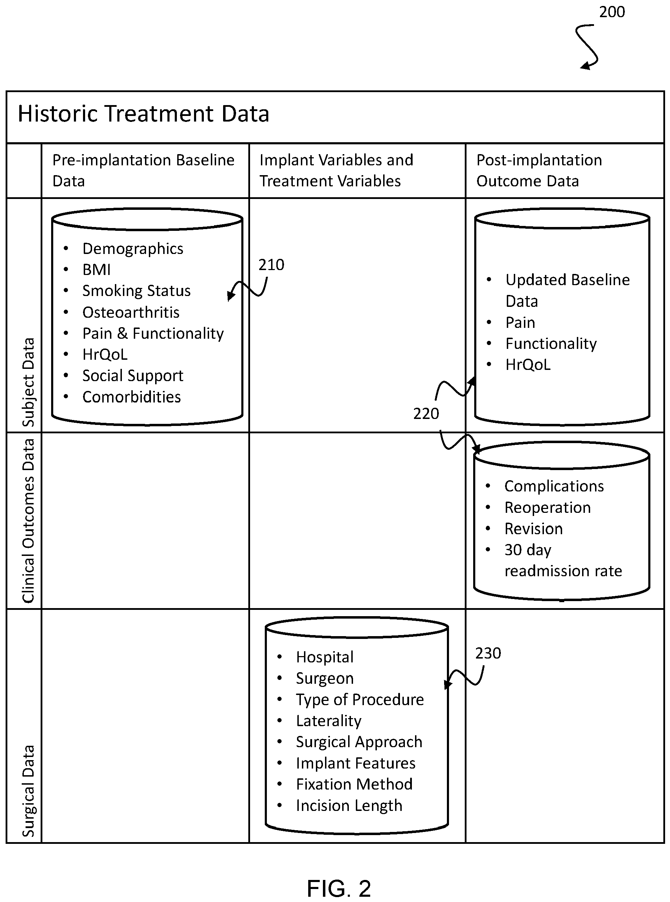

Each of the plurality of implant treatment types may be associated with one or more implant variables. In an embodiment, this may include a surgical approach, and implant type related variables. In addition, each of the plurality of implant treatment types may be associated with one or more treatment variables. In an embodiment, this may include healthcare system related variables. In this way, there may be a large number of different implant treatment types, as each combination of implant variables and/or treatment variables give rise to another implant treatment option. More information regarding implant variables and treatment variables will be given in relation to Fig. 2 below.

The historic treatment data may be data collected from historic subjects, supplemented by clinical outcomes data and surgical data (such as implant features and surgical technique). This data may be obtained from national registries, such as the Dutch Arthroplasty Register (LROI), which contains information on orthopaedic prosthesis procedures in the Netherlands in 2017. Generally, national registries collect data on implant characteristics, surgical techniques, survival of implants, patient characteristics and patient experiences. The historic treatment data is related to a plurality of different implant treatment types.

Further, the historic treatment data may comprise a plurality of pre-implantation baseline data and a plurality of post-implantation outcome data. The pre-implantation baseline data may be information about a historic subject, and may specifically include information including a diagnosis, a pain factor, a functionality factor, a quality of life factor, subject lifestyle data, and subject demographic data. The post-implantation outcome data may be information about the outcome of an implant treatment type on a historic subject, and may specifically include a pain factor, a functionality factor, a quality of life factor, a complication rate, a reoperation rate, and a 30 day readmission rate. More information regarding the historic treatment data will be given in relation to Fig. 2 below.

The modelling module 110 may further comprise a classification module 140. The classification module 140 may be configured to categorize the plurality of pre-implantation baseline data into a plurality of pre-implantation baseline classes. As a result, many classes of subjects pre-implantation may be provided, meaning that it is simpler to interpret the data as there may be many thousands of different historic subjects. The classification module 140 may be further configured to categorise the plurality of post-implantation outcome data into a plurality of post-implantation outcome classes.

The classification module 140 may be further configured to perform the categorisation of historic treatment data into the pre-implantation baseline classes and post-implantation outcome classes according to at least two variables.

In an embodiment, the two variables may be a pain factor, and a functionality factor. In this case, there may be four pre-implantation baseline classes including low-pain and low-functionality, low-pain and high-functionality, high-pain and low-functionality, and high-pain and high-functionality. In other words, historic treatment data which relates to subjects which undergo a certain treatment type, and which had similar levels of pain and functionality pre-implantation, may be grouped into the same pre-implantation baseline class. Additionally, in this case, the four post-implantation outcome classes may be the same as the pre-implantation baseline classes. Similarly, historic treatment data which relates to subjects which undergo a certain treatment type, and which reported similar levels of pain and functionality post-implantation, may be grouped into the same post-implantation outcome class.

In other words, the classification module 140 may construct a first cluster/latent class model for the pre-implantation baseline data, implant variables and treatment variables. The classification module 140 may further construct a second cluster/latent class model for post-implantation outcome data, implant variables and treatment variables. If there are multiple treatment options (e.g. different hospitals, surgeons, implants) each treatment variable may be used to construct hierarchical outcome class models. For example, there may be a class model for each hospital, or for each surgeon within a department.

The outcome prediction models may be obtained by the modelling module 110 by calculating a plurality of probabilities that a subject associated with each of the pre-implantation baseline classes will be associated with each of the post-implantation outcome classes after receiving each implant treatment type. This may make a mapping, giving a percentage chance that a subject in a given pre-implantation baseline class may be associated with a post-implantation outcome class due to a received implant treatment type. This may be performed for each pre-implantation baseline class, and for each different implant treatment type.

One method to perform this calculation of probabilities may be based on a logistic regression of the historic treatment data. However, a person skilled in the art would understand that many methods may fulfil this role, such as a machine learning method, or other statistical method.

In other words, for each possible trajectory describing the transition of a subject from a pre-implantation baseline class to a post-implantation outcome class, an outcome prediction model may be constructed to estimate the transition probability, for example by logistic regression.

The data processing module 120 is configured to receive pre-implantation baseline data of the subject. This may be received from the subject or a surgeon/doctor via the interface module 130, from medical records of the patient, or by other means.

The data processing module 120 is further configured to determine predicted subject treatment outcomes from each outcome prediction model based on the pre-implantation baseline data of the subject. To paraphrase, the data processing module 120 generates the predicted treatment outcomes of the outcome prediction models, determining which predicted treatment outcomes are relevant to the subject based on the pre-implantation baseline data of the subject.

This may be achieved by identifying the pre-implantation baseline class which most closely describes the subject based on the pre-implantation baseline data of the subject. As the pre-implantation baseline classes are generated by the classification module 140 based on pre-implantation baseline data of historic subjects, matching may be achieved by the same, or similar variables.

The data processing module 120 may then be configured to transmit only the post-implantation outcome classes and associated probabilities which are associated with the identified pre-implantation baseline class to the interface module 130. In this way, all of the information of the outcome prediction models may be filtered by the data processing module 120, leaving only information which is relevant to the subject to be sent to the interface module 130. The information which is relevant to the subject may be the plurality of probabilities that the subject will transition to the plurality of post-implantation outcome classes, for each of the plurality of treatment types.

The interface module 130 is configured to output the predicted treatment outcomes of the subject.

This may include each of the implant treatment types, and the probability that the subject will be associated with each post-implantation outcome class due to each implant treatment type.

Alternatively, the interface module 130 may be configured to obtain a subject preference profile of the subject. Further, the interface module 130 may be configured to determine one or more preferred implant treatment types based on a comparison between the subject preference profile and the probability that the subject will be associated with each post-implantation outcome class due to each of the implant treatment types. The interface module 130 may then be configured to output the one or more of the implant treatment types, and the probability that the subject will be associated with each post-implantation outcome class due to each of the one or more implant treatment types. For example, the patient preference profile may be that a decreased pain factor is the priority, while an increased functionality is not. In such a case, implant treatment types with an associated relatively high probability of transitioning the subject to a post-implantation outcome class with an associated a low pain factor may be determined to be a preferred implant treatment type. Meanwhile, implant treatment types with an associated relatively high probability of transitioning the subject to a post-implantation outcome class with an associated a high functionality factor, and a high pain factor, may be disregarded.

The interface module 130 may output the predicted treatment outcomes to the subject and a doctor/surgeon. The interface module 130 may display a corresponding best treatment option, and possible trajectories in respect to post-implantation outcome classes for the subject.

In an embodiment, the implant treatment type may be an implantable device for a hip replacement surgery. In this case, the historic treatment data comprises data relating to historic hip replacement surgeries.

Fig. 2 shows a schematic of the historic treatment data 200 in accordance with the invention. The data may be collected from patients, supplemented by clinical outcomes data and surgical data such as implant features and surgical technique, surgeon characteristics and healthcare system characteristics. The data may also be obtained from national registries.

The collection of the data may be performed by existing solutions, including but not limited to VitalHealth QuestManager, or similar solutions, for the collection of PROMS data, electronic health records for the clinical data, and financial analytics solutions for claims data.

The historic data may comprise pre-implantation baseline data 210, which may include one or more of the following:
Diagnosis. For example, the most common reason for hip replacement surgery is osteoarthritis. Other conditions that can cause hip joint damage include rheumatoid arthritis, a hip fracture, septic arthritis, ankylosing spondylitis and disorders that cause unusual bone growth (bone dysplasias).

ASA classification. The ASA physical status classification system is a system for assessing the fitness of subjects before surgery.

Charnley score. The Charnley classification system is a clinical classification system for assessing the co-morbidities of the subject.

Age.

Gender.

Smoking status, or an indication of the smoking status of the patient. A classification such as current smoker, ex-smoker but less than 12 months, ex-smoker more than12 months, never smoker.

Living condition. Living alone, with family, or in a nursing home or other facility.

Educational attainment. The highest level of education completed by the patient.

BMI. Body mass index.

Medical and musculoskeletal comorbidities. Presence of cancer, depression, diabetes, disease of the nervous system, heart disease, hypertension, kidney disease, liver disease, lung disease, peripheral vascular disease, rheumatoid arthritis or other arthritis, spinal disease.

Joint specific history. History or finding of trauma or injury, congenital or developmental disorders, or other joint disorders.

### Joint specific surgical history.

Laterality of affected j oint. Indication of which j oint(s) is (are) affected.

Physical activity level. Indication of the amount of physical activity the patient carries out. Can be collected using an available validated instrument or via a simple question such as "In a typical week, how much time do you spend doing physical activity (any activity that makes you breath hard, feel warm, and feel your heart beat faster. Examples of physical activity are walking, bicycling, and dancing and also housecleaning and gardening)?"

NRS rest. Pain at rest measured by means of a Numeric Rating Scale.

NRS activity. Pain during activity measured by means of a Numeric Rating Scale.

EQ5D index score. EQ-5D is a standardized instrument to measure health-related quality of life that can be used in a wide range of health conditions and treatments. The EQ-5D consists of a descriptive system and the EQ VAS. The descriptive system comprises five dimensions: mobility, self-care, usual activities, pain/discomfort and anxiety/depression. The scores on these five dimensions can be presented as a health profile or can be converted to a single summary index number (utility) reflecting preference compared to other health profiles.

EQ5D VAS score. The EQ VAS records the patient's self-rated health on a vertical visual analogue scale.

HOOS-PS score. The short form of the Hip disability and Osteoarthritis Outcome Score (HOOS-PS). HOOS-PS is a survey that gives an indication for the level of function in performing usual daily activities and higher level activities. The measure is scored by summing the responses to the 5 items of the HOOS-PS and converting this raw sum to the Rasch-based interval score provided in the HOOS-PS user guide.

Oxford Hip score. The Oxford Hip Score (OHS) is a short 12-item subject-reported PRO specifically designed and developed to assess function and pain with subject undergoing hip replacement surgery.

The historic data may further comprise post-implantation outcome data 220, which may include one or more of the following:
NRS rest.
NRS activity.
HOOS-PS.
EQ-5D index score.
EQ-5D VAS.
Oxford Hip score.
Functionality factor.
HrQoL. Health related quality of life.
Revision
Reoperation
30day readmissions
Complications

Each implant treatment may be associated with one or more implant variables 230, and/or one or more treatment variables 240. The implant variables 230 may include one or more of the following:
Surgical approach. Posterolateral, straight lateral, anterior, anterolateral, or other.
Length of incision.
Fixation method.
Laterality.
Bonegraft. No bonegraft, autograft, allograft, or combination of both.

Implant features. For example, for hip replacement surgery:
Type of acetabular component. Press-fit, cemented, or screw cup.

Acetabular component material. For cemented: standard PE, cross linked PE, stainless steel, or cobalt chrome. For uncemented: titanium, stainless steel, tantalum, or cobalt chrome.

Inlay material. Cross linked PE, ceramics, standard PE, or cobalt chrome.

Femur component material. Titanium, cobalt chrome, or stainless steel.

Femoral head component material. Ceramic, cobalt chrome, oxidized zirconium, or stainless steel.

Femoral head component diameter.

Classification of the acetabulum component. Classification of the component on the NOV-classification list for total hip implants (1a, 1b, 2).

Classification of the femur component. Classification of the component on the NOV-classification list for total hip implants (1a, 1b, 2).

Articulation. Ceramics on PE, metal on PE, ceramics on ceramics, oxidized zirconium on PE, metal on metal.

The treatment variables 240 may include one or more of the following:
Surgeon-related variables:
Years of experience.

Average number of implantation surgeries per year.

Total number of implantation surgeries using a surgical approach. For each of the surgical approaches, a count of the number of implantation surgeries performed applying the different surgical approaches.

Total number of implantation surgeries using an implant. For each implant type, a count of the number of implantation surgeries performed using the specific implant type.

Healthcare system-related variables:
Type of hospital. General, private, or teaching.
Reputation of hospital.

The pre-implantation baseline data 210, post-implantation outcome data 220, implant variables 230, and treatment variables 240 are not restricted to those variables listed. Indeed, different measures of pain and functionality for example, and different implant feature variables, will be obvious to those skilled in the art for different implantation procedures such as hip replacements, knee replacements, stents and defibrillators.

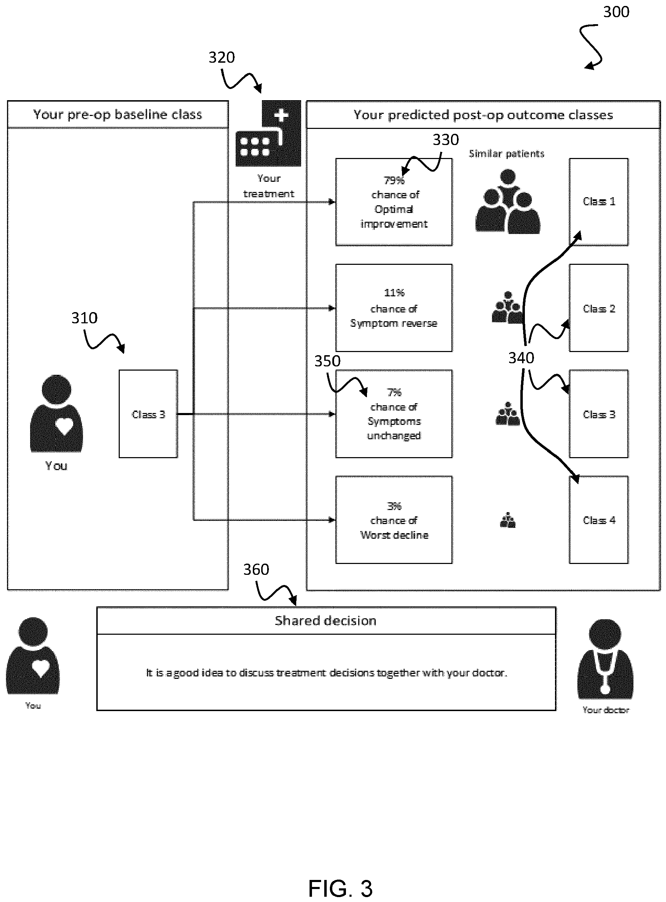

Fig. 3 shows an example output of the interface module in the form of a user interface 300 in accordance with an embodiment of the invention. This user interface may be presented to the subject and a doctor/surgeon, such that they may observe the corresponding best treatment option and possible trajectories in respect to post-implantation outcome classes.

In this example, the subject has been found to most closely match pre-implantation baseline class 3 by the data 310. The user interface has been configured to present only the implant treatment type 320 which most closely matches the subject preference profile of the subject. The relevant probabilities 330, calculated by the modelling module and filtered by the data processing module, associated with each of the four post-implantation outcome classes 340 is shown, with a brief description 350 of what each of these post-implantation outcome classes mean for the subject.

In other words, the user interface shows a sample visualization where the transition probabilities are indicted by how many similar subjects had the expected outcome starting from the same pre-implantation baseline class as the subject. Finally, a recommendation to discuss the treatment decision together with an accountable provider may be presented 360.

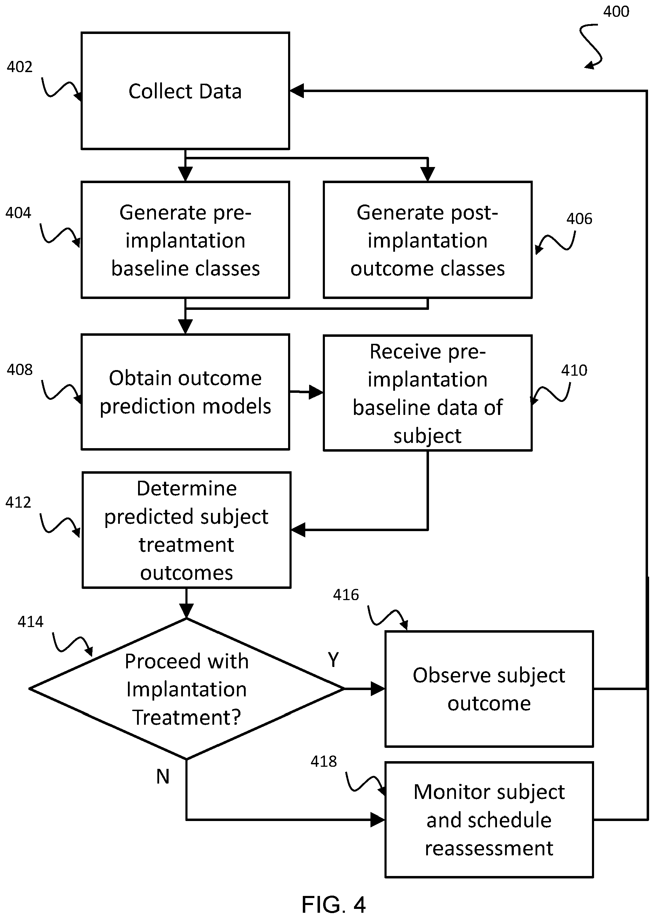

Fig. 4 is a flowchart depicting a computer-implemented method for aiding decision making in selecting an implant treatment for a subject in accordance with an embodiment of the invention.

At step 402, historic treatment data is obtained. This may be obtained from a variety of sources, such as directly from patients, or from national registries. Alternatively, the collection of the data may be performed by existing solutions, including but not limited to VitalHealth QuestManager, or similar solutions, for the collection of PROMS data, electronic health records for the clinical data, and financial analytics solutions for claims data.

This historic treatment data may be stored in such a way as to abide by the necessary legal and privacy requirements. In this example, the historic treatment data comprises plurality of pre-implantation baseline data and a plurality of post-implantation outcome data.

At step 404, the pre-implantation baseline data is classified into a plurality of pre-implantation baseline classes. In other words, pre-implantation baseline classes are generated from a cluster/latent class model of the pre-implantation baseline data.

Similarly, at step 406, the post-implantation outcome data is classified into a plurality of post-implantation outcome classes. In other words, post-implantation outcome classes are generated from a cluster/latent class model of the post-implantation outcome data.

At step 408, a plurality of outcome prediction models are obtained. Each of the outcome prediction models are based on the historic treatment data, and each outcome prediction model is associated with one of a plurality of different implant treatment types. Each outcome prediction model is used to predict treatment outcomes due to the associated implant treatment type.

In this case, each outcome prediction model is obtained by calculating a plurality of probabilities that a subject associated with each of the pre-implantation baseline classes will be associated with each of the post-implantation outcome classes after receiving the implant treatment type associated with the outcome prediction model. This may be calculated by a logistic regression of the historic treatment data, or any other appropriate method using the historic treatment data. In this way, for each implant treatment type there would exist a outcome prediction model that maps the probability that a subject in any of the pre-implantation baseline classes may transition to each of the post-implantation outcome classes due to the implant treatment type.

To take an example, Table 1 below shows when four pre-implantation baseline classes, and four post-implantation outcome classes may be formed in steps 404 and 406 respectively. The classification of both sets of classes was based upon two variables. Namely, a pain factor and a function factor. Further, the Table 1 represents the percentage of patients in a given pre-implantation baseline class, and the percentage of patients in a given post-implantation outcome class due to a given implant treatment type.

**Table 1**

| **Class** | **Time** | **Class Description** | **% patients** |
|---|---|---|---|
| Class 1 | pre-implantation | Little pain & high function | 5% |
| Class 2 | | Little pain & poor function | 4% |
| Class 3 | | High pain & high function | 75% |
| Class 4 | | High pain & poor function | 16% |
| | | | |
| Class 1 | post-implantation | Little pain & high function | 74.4% |
| Class 2 | | Little pain & poor function | 11.9% |
| Class 3 | | High pain & high function | 8.2% |
| Class 4 | | High pain & poor function | 5.5% |

Table 2 below represents the probabilities that a patient in a given pre-implantation baseline class may transition to a post-implantation outcome class, based on a logistic regression of the historic treatment data, relevant to the data represented in table 1. As such, Table 2 represents the form of an outcome prediction model.

**Table 2**

| **Baseline class** | **Outcome class** | **% baseline class patients per trajectory** | **Outcome trajectory description** |
|---|---|---|---|
| 1 | 1 | 85% | Unchanged |
| 1 | 2 | 5.5% | Decline |
| 1 | 3 | 5.5% | Decline |
| 1 | 4 | 4% | Worst Decline |
| 2 | 1 | 65% | Optimal Improvement |
| 2 | 2 | 25% | Unchanged |
| 2 | 3 | 6% | Symptom reverse |
| 2 | 4 | 4% | Worst Decline |
| 3 | 1 | 79% | Optimal Improvement |
| 3 | 2 | 11% | Symptom reverse |
| 3 | 3 | 7% | Unchanged |
| 3 | 4 | 3% | Worst Decline |
| 4 | 1 | 52% | Optimal Improvement |
| 4 | 2 | 15% | Improvement |
| 4 | 3 | 15% | Improvement |
| 4 | 4 | 18% | Unchanged |

At step 410, pre-implantation baseline data of the subject is received. This may be received from the subject or a surgeon/doctor via the interface module, from medical records of the patient, or by other means.

At step 412, predicted subject treatment outcomes are determined from each outcome prediction model based on the pre-implantation baseline data of the subject. In other words, the predicted treatment outcomes of the outcome prediction models are generated by determining which predicted treatment outcomes are relevant to the subject based on the pre-implantation baseline data of the subject.

This determination may be made by identifying the pre-implantation baseline class which most closely describes the subject based on the pre-implantation baseline data of the subject. In line with the example above, if pre-implantation baseline data of the subject indicates that the subject has high pain and poor function, then the subject is most closely aligned with pre-implantation baseline class 4, and predicted subject treatment outcomes would be the last four rows of table 2 - each of which related to pre-implantation baseline class 4.

At step 414, the predicted subject treatment outcomes are outputted. At this point the subject, in consultation with their doctor/surgeon, may decide whether to proceed with an implantation treatment. If it is decided to proceed with an implantation treatment, then step 416 is executed, if not then step 418 is executed.

At step 416, the treatment outcome of the subject is monitored. This can then be used as past of the historic treatment data for future subjects.

At step 418, the subject is monitored, and a reassessment scheduled.

A single processor or other unit may fulfil the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (100) for aiding decision making in selecting an implant treatment for a subject, comprising:
a modelling module (110) configured to obtain a plurality of outcome prediction models, wherein each outcome prediction model is based on historic treatment data of a plurality of different implant treatment types, wherein each outcome prediction model is associated with one of the plurality of different implant treatment types, and wherein each outcome prediction model is used to predict treatment outcomes due to the associated implant treatment type;
a data processing module (120) configured to receive pre-implantation baseline data of the subject, and further configured to determine predicted subject treatment outcomes from each outcome prediction model based on the pre-implantation baseline data of the subject; and
an interface module (130) configured to output the predicted subject treatment outcomes of the subject.

2. The system of claim 1, wherein the historic treatment data comprises a plurality of pre-implantation baseline data and a plurality of post-implantation outcome data, and wherein the modelling module (110) further comprises:
a classification module (140) configured to categorise the plurality of pre-implantation baseline data into a plurality of pre-implantation baseline classes, and to categorise the plurality of post-implantation outcome data into a plurality of post-implantation outcome classes.

3. The system of claim 2, wherein the classification module (140) is configured to categorise the plurality of pre-implantation baseline data into the plurality of pre-implantation baseline classes, and to categorise the plurality of post-implantation outcome data into the plurality of post-implantation outcome classes, according to at least two variables, and
optionally wherein the at least two variables comprise a pain factor and a functionality factor.

4. The system of claim 2 or 3, wherein obtaining the plurality of outcome prediction models comprises calculating a plurality of probabilities that a subject associated with each of the pre-implantation baseline classes will be associated with each of the post-implantation outcome classes after receiving each implant treatment type.

5. The system of claim 4, wherein calculating the plurality of probabilities is based on a logistic regression of the historic treatment data.

6. The system of claim 4 or 5, wherein the data processing module (120) is configured to identify the pre-implantation baseline class which most closely describes the subject based on the pre-implantation baseline data of the subject, and wherein the data processing module is further configured to transmit only the post-implantation outcome classes and associated probabilities which are associated with the identified pre-implantation baseline class to the interface module.

7. The system of claim 6, wherein the interface module (130) is configured to output each of the implant treatment types, and the probability that the subject will be associated with each post-implantation outcome class due to each implant treatment type.

8. The system of claim 6, wherein the interface module (130) is further configured to:
obtain a subject preference profile of the subject;
determine one or more preferred implant treatment types based on a comparison between the subject preference profile and the probability that the subject will be associated with each post-implantation outcome class due to each of the preferred implant treatment types; and
output the one or more preferred implant treatment types, and the probability that the subject will be associated with each post-implantation outcome class due to each of the one or more preferred implant treatment types.

9. The system of any preceding claim, wherein each of the plurality of implant treatment types is associated with one or more implant variables, wherein the one or more implant variables include:
a surgical approach; and
implant type related variables.

10. The system of any preceding claim, wherein each of the plurality of implant treatment types is further associated with one or more treatment variables, wherein the one or more treatment variables include:
healthcare system related variables; and
surgeon related variables.

11. The system of any preceding claim, wherein the pre-implantation baseline data includes one or more of:
a diagnosis;
a pain factor;
a functionality factor;
a quality of life factor;
subject lifestyle data; and
subject demographic data.

12. The system of any preceding claim, wherein the post-implantation outcome data includes one or more of:
a pain factor;
a functionality factor;
a quality of life factor;
a complication rate;
a reoperation rate; and
a 30 day readmission rate.

13. The system of any preceding claim, wherein the implant treatment type is an implantable device for a hip replacement surgery, and wherein the historic treatment data comprises data relating to historic hip replacement surgeries.

14. A computer-implemented method for aiding decision making in selecting an implant treatment for a subject, comprising:
obtaining (408) a plurality of outcome prediction models, wherein each outcome prediction model is based on historic treatment data of a plurality of different implant treatment types, wherein each outcome prediction model is associated with one of the plurality of different implant treatment types, and wherein each outcome prediction model is used to predict treatment outcomes due to the associated implant treatment type;
receiving (410) pre-implantation baseline data of the subject;
determining (412) predicted subject treatment outcomes from each outcome prediction model based on the pre-implantation baseline data of the subject; and
outputting the predicted subject treatment outcomes of the subject.

15. A computer program comprising computer program code means adapted, when said computer program is run on a computer, to implement the method of claim 14.
